# EUROPÄISCHE PATENTSCHRIFT

(11) **EP 1 005 849 B1**
(45) Veröffentlichungstag und Bekanntmachung des Hinweises auf die Patenterteilung: **19.09.2001**
(21) Anmeldenummer: 99120517.0
(22) Anmeldetag: 15.10.1999
(51) Int. Cl.: A61K 7/00, A61K 7/06, A61K 7/48

(54) **Zweiphasige Zusammensetzung zur Haar- oder Kopfhautbehandlung**
Two-phase composition for treating the hair or the scalp
Composition à deux phases pour le traitement des cheveux ou du cuir chevelu

(30) Priorität: 03.12.1998 DE 19855767
(43) Veröffentlichungstag der Anmeldung: 07.06.2000
(73) Patentinhaber: Wella Aktiengesellschaft, 64274 Darmstadt (DE)
(72) Erfinder: Koschik, Achim, 64297 Darmstadt (DE); Breda, Carina, 64354 Reinheim (DE); Jahedshoar, Mehrdad, Calabasas, CA 91302 (US); Eicken, Ulrich, 1700 Fribourg (CH); Jungo, Sybille, 1723 Marly (CH); Bormuth, Hiltrud, 64658 Fürth (DE)

(56) Entgegenhaltungen:
- EP-A- 0 490 750
- EP-A- 0 870 495
- DE-A- 2 259 756
- US-A- 4 767 741
- US-A- 5 725 844

## Beschreibung

Gegenstand der Erfindung ist eine Zusammensetzung gebildet aus mindestens zwei nicht miteinander mischbaren flüssigen Phasen, wobei eine der Phasen in Form von Tropfen vorliegt, welche von einer Größe sind, die mit bloßem Auge gut sichtbar ist, d.h. einen Durchmesser von mindestens etwa 1 mm besitzen, zur Behandlung von Haaren und/oder der Kopfhaut.

Die Haare und die Kopfhaut werden durch Einwirkungen verschiedener Art in ihren physikalischen, chemischen und morphologischen Eigenschaften negativ beeinflußt. So wird durch kosmetische Behandlungen wie zum Beispiel häufiges Bleichen, Dauerwellen und Färben und selbst bereits durch häufiges Waschen der Haare mit entfettenden Tensiden sowie durch Klimaeinflüsse wie Feuchte- und Temperaturunterschiede oder durch die intensive Einwirkung von Sonnenlicht die Haarstruktur geschädigt. Das Haar wird spröde und verliert seinen natürlichen Glanz. Das so geschädigte Haar lädt sich beim Kämmen und Bürsten elektrostatisch auf und die aufgerauhte Haaroberfläche führt durch Verfilzungen und Verknotungen zu einer schlechten Kämmbarkeit und Entwirrbarkeit des Haares.

Dieser negative Haarzustand kann mit Hilfe von Haarpflegemitteln, wie z.B. Haarkurmitteln verbessert werden. Haarkurmittel mit einer pflegenden und kämmbarkeitsverbessernden Wirkung sind daher für die moderne Haarpflege von großer Bedeutung. Haarkurmittel liegen üblicherweise in Form von Emulsionen oder Suspensionen vor, die Fettalkohole, Wachse und Öle sowie synthetische, organische, anionische, kationische, amphotere, nichtionische, überwiegend jedoch kationische Emulgatoren enthalten.

Die bisherigen Haarkurmittel sind nicht in allen Punkten völlig zufriedenstellend, so daß ein fortgesetzter Bedarf an Verbesserungen besteht. Insbesondere wäre ein Haarkurmittel von Interesse, welches ohne synthetische organische Emulgatoren auskommt, da diese eine zu stark entfettende oder auch in gewissem Umfang eine hautreizende Wirkung besitzen können. Insbesondere bei kationischen Emulgatoren sind den verwendbaren Mengen enge Grenzen gesetzt. Mit einer hohen Konzentration würden zwar vorteilhafte Emulsionseigenschaften erzielt werden können, andererseits aber nachteilige Wirkungen auf Kopfhaut und Haaren hervorgerufen. So kann vor allem bei Anwendung auf normalem, ungeschädigten Haar eine zu starke Pflegewirkung eintreten, die sich in einer Belastung des Haares sowohl im nassen als auch im trockenen Zustand zeigt. Bei einer zu starken Pflegewirkung kann auf dem Haar sogar ein Umkehreffekt eintreten, wobei das Haar nach der Behandlung stumpf und schwerer kämmbar wird als vor der Behandlung.

Geringe Mengen an kationischen Emulgatoren verursachen zwar keine Hautschäden, lassen aber andererseits keine brauchbaren Emulsionen entstehen und entfalten nur eine unbefiedigende Pflegewirkung. Darüberhinaus haben kationische Emulgatoren die zusätzlichen Nachteile, daß sie schleimhautreizend und biologisch nicht oder nur schlecht abbaubar sind.

Außerdem sind Haartonika oder Haarwässer auf Basis von alkoholischen oder wäßrig-alkoholischen Lösungen bekannt. Diese Mittel dienen entweder dazu, dem Haar eine angenehme Duftnote zu verleihen, sofern sie lediglich Duftstoffe enthalten oder sie dienen dazu, Kopfhaut und Haar gesund zu erhalten oder deren normale Funktion wieder herzustellen. In diesem Fall enthalten sie entsprechende, in dem alkoholischen oder wäßrig-alkoholischen Medium gelöste Wirkstoffe.

Aus der DE 2 259 756 sind zweiphasige, flüssige kosmetische Zubereitungen bekannt, die aus einer wässrigen Phase und einer Ölphase bestehen, feinteilige, in beiden Phasen unlösliche feste Dispergatoren enthalten und wobei die Ölphase in Form von Kügelchen dispergiert ist.

Es stellte sich die Aufgabe, ein Mittel zur Verfügung zu stellen, welches die Anforderungen, die an eine ölartige Stoffe enthaltende Haarkur einerseits und an ein Haartonikum oder Haarwasser andererseits gestellt werden, gleichermaßen erfüllt und zudem ohne organische, insbesondere ohne kationische Emulgatoren auskommt. Das Mittel soll außerdem in einer optisch ansprechenden Form vorliegen und leicht auf dem Haar verteilbar sein.

Es wurde nun gefunden, daß eine Zusammensetzung zur Behandlung von Haaren und/oder der Kopfhaut die gestellten Anforderungen erfüllt, wenn sie mindestens zwei nicht miteinander mischbare flüssige Phasen enthält, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe Flüssigkeit enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm, vorzugsweise von 1 bis 10 mm besitzen,
(C) die Zusammensetzung mindestens einen feinverteilten, pulverförmigen, in der Zusammensetzung unlöslichen, anorganischen Feststoff als Dispergator enthält,
(D) die Zusammensetzung mindestens ein filmbildendes, haarfestigendes Polymer enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet. Die Tropfen sind in Größe, Form und Aussehen vorzugsweise von perlenartiger Erscheinung.

Eine besonders gute Verteilbarkeit auf dem Haar wird erreicht, wenn die Zusammensetzung mit einer Vorrichtung zum Versprühen kombiniert wird. Das erfindungsgemäße Haarbehandlungsmittel liegt dann in Form eines Non-Aerosol-Haarsprays vor und wird mit Hilfe einer geeigneten mechanisch betriebenen Sprühvorrichtung versprüht. Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in dem das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden. Gegenstand der Erfindung ist daher auch ein Mittel zur Behandlung von Haaren oder der Kopfhaut, bestehend aus einer Zusammensetzung und einer Vorrichtung zum Versprühen der Zusammensetzung, wobei die Zusammensetzung mindestens zwei nicht miteinander mischbare flüssige Phasen enthält, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe Flüssigkeit enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm besitzen,
(C) die Zusammensetzung mindestens einen feinverteilten, pulverförmigen, in der Zusammensetzung unlöslichen, anorganischen Feststoff als Dispergator enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet.

Eine besonders geringe Belastung des behandelten Haares wird erreicht, wenn die hydrophobe Phase mindestens eine hydrophobe, flüssige, leicht flüchtige Substanz enthält, die vorzugsweise ausgewählt ist aus flüchtigen Silikonen und flüchtigen Kohlenwasserstoffen. Ein weiterer Gegenstand der Erfindung ist daher eine Zusammensetzung zur Behandlung von Haaren oder der Kopfhaut, enthaltend mindestens zwei nicht miteinander mischbare flüssige Phasen, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe, flüssige, leicht flüchtige Substanz, vorzugsweise ausgewählt aus Silikonen und Kohlenwasserstoffen, enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm besitzen,
(C) die Zusammensetzung mindestens einen feinverteilten, pulverförmigen, in der Zusammensetzung unlöslichen, anorganischen Feststoff als Dispergator enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet.

In einer bevorzugten Ausführungsform besteht die hydrophobe Phase aus einer Kombination aus mindestens einem leicht flüchtigen Kohlenwasserstoff und mindestens einer schwer flüchtigen, hydrophoben Flüssigkeit. Leicht flüchtig im Sinne dieser Erfindung sind Stoffe,die bei 100°C innerhalb von 20 Minuten restlos von einer in dem Stoff getränkten Haarsträhne verflüchtigt. Bevorzugt sind Stoffe, die bei Normaldruck einen Siedepunkt von unter 250°C aufweisen. Schwer flüchtig im Sinne der Erfindung sind solche Stoffe, die nicht leicht flüchtig gemäß der obigen Definition sind. Geeignete leicht flüchtige Substanzen sind leichtflüchtige Paraffine, leichtflüchtige Isoparrafine oder leichtflüchtige Silikonöle. Bevorzugt sind verzweigte oder unverzweigte Kohlenwasserstoffe mit 6 bis 14 C-Atomen, vorzugsweise mit 8 bis 12 C-Atomen wie z.B. Dekan oder Dodekan und deren Isomere sowie lineare oder cyclische Dimethylpolysiloxane wie z.B. Dimethicone oder Cyclomethicone.

Als Dispergatoren kann die Zusammensetzung feinverteilte, pulverförmige, in der Zusammensetzung unlösliche, anorganische Feststoffe enthalten, die auch unter der Bezeichnung Pickering-Emulgatoren bekannt sind. Bekannt sind z.B. Talkum, Calciumcarbonat, Magnesiumcarbonat, Bariumsulfat, Aluminiumhydroxid, Hydroxylapatit, Tricalciumphosphat und Calciumoxalat. Geeignet sind weiterhin Silica, Mica und Titandioxid. Zur Erzielung von Farb- oder Perlglanzeffekten können die anorganischen Dispergatoren mit weiteren, in der Zusammensetzung unlöslichen Feststoffen wie z.B. Farb- oder Perglanzpigmenten, insbesondere solchen auf Glimmerbasis, vermischt eingesetzt werden. Die Einsatzmenge der Dispergatoren beträgt vorzugsweise von 0,01 bis 1 Gewichtsprozent, besonders bevorzugt von 0,1 bis 0,6 Gewichtsprozent. Die Dispergatoren können aber auch schon ab 0,001 Gew.% und in Konzentrationen bis zu 2 Gew.% eingesetzt werden. Der Gehalt an Pigmenten kann 0,001 bis 0,5 Gewichtsprozent betragen.

Als Lösungsmittel für die erste, kontinuierliche Phase der Zusammensetzung kommen in erster Linie Wasser, einwertige verzweigte oder unverzweigte Alkohole mit vorzugsweise 1 bis 4 Kohlenstoffatomen und mehrwertige Alkohole mit vorzugsweise 1 bis 4 Kohlenstoffatomen oder Gemische der genannten Lösungsmittel in Betracht. Bevorzugte einwertige Alkohole sind z.B. Ethanol, n-Propanol und Isopropanol. Bevorzugte mehrwertige Alkohole sind Ethylenglykol, Propylenglykol und Glycerin, von denen das Glycerin besonders bevorzugt ist. Vorteilhaft ist insbesondere ein hydrophiles Lösungsmittelgemisch aus Wasser, mindestens einem einwertigen verzweigten oder unverzweigten Alkohol mit 1 bis 4 Kohlenstoffatomen und mindestens einem mehrwertigen Alkohol.

Die kontinuierliche, hydrophile Phase macht vorzugsweise 50 bis 95, besonders bevorzugt 60 bis 90 Gewichtsprozent der Gesamtzusammensetzung aus. Die einwertigen Alkohole sind vorzugsweise in einer Menge von 60 bis 95, besonders bevorzugt von 70 bis 90 Gewichtsprozent enthalten. Die mehrwertigen Alkohole sind vorzugsweise in einer Menge von 0 bis 30, besonders bevorzugt von 5 bis 20 Gewichtsprozent enthalten. Wasser wird vorzugsweise in einer Menge von 0 bis 30, besonders bevorzugt von 5 bis 20 Gewichtsprozent eingesetzt und kann gelöste anorganische Salze wie z.B. Calciumchlorid, Natriumchlorid oder Natriumsulfat enthalten.

In einer bevorzugten Ausführungsform enthält die Zusammensetzung zusätzlich mindestens ein filmbildendes und haarfestigendes Polymer, sofern die Tröfpchenstruktur dadurch nicht beeinträchtigt wird. Hierdurch wird ein Mittel erhalten, welches zusätzlich zu der pflegenden Wirkung auf Haar und Kopfhaut auch einen Stylingeffekt aufweist, d.h. dem Haar besseren Halt oder mehr Volumen verleiht. Das haarfestigende Polymer ist vorzugsweise in der hydrophilen Phase löslich.

Das haarfestigende Polymer ist ausgewählt aus synthetischen oder natürlichen, nichtionischen, kationischen, anionischen oder amphoteren Polymeren. Ein derartiges Polymeres, das in Mengen von 0,01 bis 30 Gewichtsprozent, vorzugsweise in Mengen von 0,5 bis 20 Gewichtsprozent im Haarbehandlungsmittel enthalten sein kann, kann auch aus einem Gemisch mehrerer Polymerer bestehen und durch den Zusatz weiterer Polymerer mit verdickender Wirkung in seinen haarfestigenden Eigenschaften noch modifiziert werden.

Unter haarfestigenden Polymeren werden erfindungsgemäß solche Polymere verstanden, die bei Anwendung in 0,01 bis 5%-iger wäßriger, alkoholischer oder wäßrig-alkoholischer Lösung in der Lage sind, auf dem Haar einen Polymerfilm abzuscheiden und auf diese Weise das Haar zu festigen.

Geeignete synthetische, nichtionische filmbildende, haarfestigende Polymere sind z.B. Homopolymere des Vinylpyrrolidons, sowie Homopolymere des N-Vinylformamids. Weitere geeignete synthetische filmbildende, nicht-ionische, haarfestigende Polymere sind z.B. Copolymerisate aus Vinylpyrrolidon und Vinylacetat, Terpolymere aus Vinylpyrrolidon, Vinylacetat und Vinylpropionat, Polyacrylamide, die beispielsweise unter den Handelsbezeichnungen Akyponine® P 191 von der Firma CHEM-Y, Emmerich, oder Sepigel® 305 von der Firma Seppic vertrieben werden; Polyvinylalkohole, die beispielsweise unter den Handelsbezeichnungen Elvanol® von Du Pont oder Vinol® 523/540 von der Firma Air Products vertrieben werden sowie Polyethylenglykol/ Polypropylenglykol-Copolymere, die beispielsweise unter den Handelsbezeichnungen Ucon® der Union Carbide vertrieben werden.

Geeignete natürliche filmbildende Polymere mit haarfestigender Wirkung sind zum Beispiel Chitosan mit einem Molekulargewicht von 20.000 bis ca. 5 Millionen g/mol. Desweiteren können verschiedene Saccharidtypen verwendet werden wie Polysaccharide oder Gemische aus Oligo-, Mono- und Disacchariden, welche beispielsweise unter dem Handelsnamen C-PUR® von der Firma Cerestar, Brüssel, vertrieben werden. Weitere geeignete, natürliche Polymere sind chinesisches Balsamharz und Cellulosederivate, z.B. Hydroxypropylcellulose mit einem Molekuklargewicht von 30.000 bis 50.000 g/mol, welche beispielsweise unter der Handelsbezeichnung Nisso Sl® von der Firma Lehmann & Voss, Hamburg, vertrieben wird. Ein weiteres natürliches Polymer ist Schellack. Schellack kann in neutralisierter Form und unneutralisiert zum Einsatz kommen.

Geeignete anionische Polymere enthalten Säuregruppen, die mit geeigneten Basen neutralisierbar sind. Die Säuregruppen sind vorzugsweise ausgewählt aus -COOH, -SO₃H, -OSO₃H, -OPO₂H und -OPO₃H₂. Carbonsäuregruppen sind besonders beorzugt. Die Säuregruppen liegen vorzugsweise zu 50 bis 100% in anionischer bzw. neutralisierter Form vor. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH und andere.

Das haarfestigende Polymer kann ein natürliches oder ein synthetisches Homo- oder Copolymer mit Säuregruppen enthaltenden Monomereinheiten sein, welches gegebenenfalls mit Comonomeren, die keine Säuregruppen enthalten, copolymerisiert ist. Geeignete Monomere sind ungesättigte, radikalisch polymerisierbare Verbindungen, welche mindestens eine Säuregruppe tragen, insbesondere Carboxyvinylmonomere. Geeignete Säuregruppen enthaltende Monomere sind beispielsweise Acrylsäure, Methacrylsäure, Crotonsäure, Maleinsäure bzw. Maleinsäureanhydrid oder deren Monoester, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte anionische Polymere sind vernetzte oder unvernetzte Vinylacetat/Crotonsäure Copolymere. Ebenso bevorzugt sind partialveresterte Copolymere zwischen Vinylmethylether und Maleinsäureanhydrid. Weitere geeignete anionische Polymere sind zum Beispiel Terpolymere aus Acrylsäure, Alkylacrylat und N-Alkylacrylamid, insbesondere Acrylsäure/Ethylacrylat/N-t-Butylacrylamid Terpolymere oder Terpolymere aus Vinylacetat, Crotonat und Vinylalkanoat, insbesondere Vinylacetat/Crotonat/Vinylneodecanoat Copolymere.

Geeignete amphotere Polymere sind solche, welche neben sauren oder anionischen Gruppen als weitere funktionelle Gruppen basische oder kationische Gruppen, insbesondere primäre, sekundäre, tertiäre oder quaternäre Amingruppen enthalten. Beispiele hierfür sind Copolymere gebildet aus Alkylacrylamid (insbesondere Octylacrylamid), Alkylaminoalkylmethacrylat (insbesondere t-Butylaminoethylmethacrylat) und zwei oder mehr Monomeren bestehend aus Acrylsäure, Methacrylsäure oder deren Ester, wie sie zum Beispiel unter dem Handelsnamen Amphomer® oder Amphomer® LV-71 der Firma NATIONAL STARCH, USA erhältlich sind. Weitere Beispiele für geeignete amphotere Copolymere sind Copolymere von Acrylsäure, Methylacrylat und Methacrylamidopropyltrimethylammoniumchlorid (INCI-Bezeichnung: Polyquaternium-47), wie sie beispielswiese von der Firma Calgon unter der Handelsbezeichnung Merquat® 2001 vertrieben werden, Copolymere aus Acrylamidopropyltrimethylammoniumchlorid und Acrylaten, wie sie beispielsweise von der Firma Stockhausen unter der Handelsbezeichnung W 37194 erhältlich sind oder Copolymere aus Acrylamid, Acrylamidopropyltrimethylammoniumchlorid, 2-Amidopropylacrylamidsulfonat und Dimethylaminopropylamin (INCI-Bezeichnung: Polyquaternium-43), wie sie beispielsweise von der Firma Societe Francaise Hoechst unter der Handelsbezeichnung Bozequat® 4000 vertrieben werden. Geeignet sind auch Polymere mit Betaingruppen tragenden Monomeren wie z.B. Copolymere aus Methacryloylethylbetain und zwei oder mehr Monomeren von Acrylsäure oder deren einfachen Estern, bekannt unter der INCI-Bezeichnung Methacryloyl Ethyl Betaine/Acrylates Copolymer.

Geeignete Polymere mit basischen Gruppen haben ein Molekulargewicht von vorzugsweise mindestens 50.000 g/mol, besonders bevorzugt von 100.000 bis 6.000.000 g/mol und enthalten stickstoffhaltige Gruppen wie zum Beispiel primäre, sekundäre oder tertiäre Amine. Die basischen Polymere können mit geeigneten, kosmetisch verträglichen Säuren teilweise oder ganz neutralisiert sein und somit in kationischer Form vorliegen. Geeignete Säuren sind z.B. Ameisensäure, Pyrrolidoncarbonsäure, Milchsäure usw. Die basische Gruppe ist entweder in der Polymerkette oder vorzugsweise als Substituent an einem oder mehreren Monomeren enthalten.

Das Polymer mit basischen Gruppen kann ein natürliches oder ein synthetisches Homo- oder Copolymer mit aminsubstituierten Monomereinheiten sowie gegebenenfalls mit nicht-basischen Comonomeren sein. Geeignete Polymere mit basischen Gruppen sind zum Beispiel Copolymere von aminsubstituierten Vinylmonomeren und nicht aminsubstituierten Monomeren. Aminsubstituierte Vinylmonomere sind zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise niedere Alkylgruppen wie zum Beispiel C1- bis C7-Alkylgruppen, besonders bevorzugt C1- bis C3-Alkylgruppen sind.

Nicht aminsubstituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Maleinsäureanhydrid, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit kationischen Gruppen enthalten vorzugsweise quaternäre Amingruppen. Die kationischen Polymere können Homo- oder Copolymere sein, wobei die quaternären Stickstoffgruppen entweder in der Polymerkette oder vorzugsweise als Substituent an einem oder mehreren Monomeren enthalten sind. Die Ammoniumgruppen enthaltenden Monomere können mit den oben genannten nicht aminsubstituierten Monomeren copolymerisiert sein. Geeignete ammoniumsubstituierte Vinylmonomere sind zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alyklvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16), quaternisiertes Vinylpyrrolidon/Dimethylaminoethylmethacrylat Copolymer (Polyquaternium-11), Homo- und Copolymere von Dimethyldiallylammoniumchlorid (Polyquaternium-6 und -7), quaternisierte Hydroxyethylcellulose (Polyquaternium-10) oder quaternisierte Guarderivate.

Von den kationischen Polymeren, die in dem erfindungsgemäßen Mittel enthalten sein können, ist zum Beispiel Polyvinylpyrrolidon/Dimethylaminoethylmethacrylat Copolymer geeignet. Weitere kationische Polymere sind beispielsweise das Copolymer aus Polyvinylpyrrolidon und Imidazoliminmethochlorid, das Terpolymer aus Dimethyldiallylammoniumchlorid, Natriumacrylat und Acrylamid, das Terpolymer aus Vinylpyrrolidon, Dimethylaminoethylmethacrylat und Vinylcaprolactam, quaternierte Ammoniumsalze aus Hydroxyethylcellulose und einem trimethylammonium-substituierten Epoxid und Vinylpyrrolidon/Methacrylamidopropyltrimethylammoniumchlorid Copolymere.

Die zweite, diskontinuierlich, tropfenförmig vorliegende Phase enthält als Lösungsmittel oder als haarpflegende Wirkstoffe hydrophobe Flüssigkeiten in einer Menge von vorzugsweise 2 bis 35, besonders bevorzugt von 5 bis 30 Gewichtsprozent bezogen auf die Gesamtzusammensetzung. Geeignete haarpflegende ölartige Flüssigkeiten sind beispielsweise pflanzlichen, synthetischen oder mineralischen Ursprungs oder deren Gemische. Geeignete Lösungsmittel sind beispielsweise lineare oder verzweigte aliphatische oder aromatische Kohlenwasserstoffe mit mindestens 5 Kohlenstoffatomen oder Silikonverbindungen wie z.B. niedrigviskose cyclische oder lineare Dimethylpolysiloxane (Silikonöle). Geeignete Bestandteile der hydrophoben Phase sind weiterhin Paraffine, Isoparaffine, Mineralöl, Paraffinöl, Pflanzenöle, Fettsäureester, Fettsäuren, Fettalkohole, leicht spreitende Öle wie Isopropylmyristat, Dicaprylether, Octyldodecanol, Jojobaöl, Phospholipide wie Lecithin, Ceramide, Silikonverbindungen oder deren Gemische. Bei den Fettsäureestern kann es sich um Ester von Fettsäuren mit kurzkettigen (C1 bis C6) Monoalkoholen oder mit Fettalkoholen oder um Mono-, Di- oder Triglyceride handeln, wie sie insbesondere in natürlich vorkommenden pflanzlichen Ölen wie Avocadoöl, Sonnenblumenöl usw. enthalten sind. Bevorzugte Fettalkohole sind linear und haben eine Kettenlänge von 8 bis 22 Kohlenstoffatomen. Bevorzugte Inhaltstoffe der hydrophoben Phase sind ausgewählt aus Paraffinen, Isoparaffinen, Mineralöl, Paraffinöl, Pflanzenölen, Fettsäureestern, Fettsäureglyceriden und Silikonverbindungen. Besonders bevorzugt sind Paraffinöle und Silikonöle.

In der hydrophoben Phase können vorteilhafterweise auch haar- und/oder hautpflegende nicht-flüssige Stoffe in gelöster Form vorliegen, beispielsweise Wachse, z.B. Frucht- oder Pflanzenwachse wie Apfelwachs, Ilexharz, Bienenwachs oder auch Lanolin, Paraffinwachs, Vaseline oder höherviskose Silikonverbindungen und Silikonharze.

Der erfindungsgemäßen Zusammensetzung können weitere, übliche kosmetische Zusatzstoffe beigefügt werden, sofern sie die Zweiphasen- und Tropfenstruktur nicht beeinträchtigen, zum Beispiel Parfümöle in einer Menge von 0,1 bis 5 Gewichtsprozent, Trübungsmittel wie Ethylenglykoldistearat in einer Menge von 0,2 bis 5 Gewichtsprozent, bakterizide und fungizide Wirkstoffe, Verdickungsmittel, pH-Puffersubstanzen, Feuchthaltemittel, Farbstoffe, Lichtschutzmittel, Antioxidantien, Komplexbildner, Antischuppenwirkstoffe sowie ferner physiologisch verträgliche Säuren, wie zum Beispiel Ameisensäure, Glyoxylsäure, Milchsäure, Weinsäure oder Zitronensäure und natürliche, modifizierte natürliche oder synthetische Polymere.

Besonders bevorzugte Zusatzstoffe sind die für ein Haartonikum bzw. für ein Haarwasser typischen Zusatzstoffe wie Duftstoffe und Wirkstoffe, z.B. Pflanzenextrakte, Vitamine, Aminosäuren, gefäßerweiternde Mittel, bakterizide Substanzen, Antischuppenstoffe und andere.

Eine bevorzugte Zusammensetzung enthält
(A) 60 bis 95 Gewichtsprozent mindestens eines einwertigen verzweigten oder unverzweigten Alkohols mit 1 bis 4 Kohlenstoffatomen,
(B) mindestens einen mehrwertigen Alkohol mit 1 bis 4 Kohlenstoffatomen in einer Menge bis maximal 30 Gewichtsprozent,
(C) Wasser in einer Menge bis maximal 30 Gewichtsprozent,
(D) 2 bis 35 Gewichtsprozent mindestens einer hydrophoben Flüssigkeit,
(E) 0,01 bis 1 Gewichtsprozent mindestens eines feinverteilten, pulverförmigen, in der Zusammensetzung unlöslichen, anorganischen Dispergators und
(F) mindestens ein filmbildendes, haarfestigendes Polymer.

Anzahl und Größe der Tröpfchen der hydrophoben Phase kann durch Verändern des Mengenverhältnisses von hydrophiler zu hydrophober Phase sowie durch Variation der Menge an Dispergator eingestellt werden. Über die Einstellung der Dichte der Phasen kann erreicht werden, daß die mit dem Dispergator umhüllten hydrophoben Tröpfchen in der kontinuierlichen, hydrophilen Phase schweben oder nahezu schweben. Eine derartige Zusammensetzung stellt eine besonders bevorzugte Ausführungsform dar. Die Dichte einer alkoholischen, z.B. einer ethanolischen Phase kann beispielsweise durch Zugabe eines hydrophilen Stoffes höherer Dichte wie z.B. Glycerin oder durch Zugabe von gegebenenfalls salzhaltigem Wasser erhöht werden. In einer bevorzugten Ausführungsform mit nahezu schwebenden Paraffintröpfchen sind 65 bis 75, besonders bevorzugt ca. 70 Gewichtsprozent Ethanol, 5 bis 15, besonders bevorzugt ca. 10 Gewichtsprozent Glycerin, 15 bis 25, besonders bevorzugt ca. 20 Gewichtsprozent Paraffinöl und 0,2 bis 0,4, besonders bevorzugt ca. 0,3 Gewichtsprozent Dispergator, insbesondere Talkum enthalten.

Die Zusammensetzung wird angewendet, indem sie unmittelbar vor der Anwendung per Hand geschüttelt wird, bis sich eine feinere Emulsion gebildet hat, die anschließend auf trockenes oder feuchtes Haar aufgetragen wird. Die Zusammensetzung kann in die Kopfhaut einmassiert werden. Anschließend wird das Haar gegebenenfalls gespült, frisiert und getrocknet.

Die nachfolgenden Beispiele sollen den Gegenstand der Erfindung näher erläutern, ohne ihn darauf zu beschränken.

### Beispiel 1: Zweiphasige Haarbehandlungsmittel mit perlenartigen Paraffintröpfchen

Es wird Talkum mit den unten angegebenen Mengen an Paraffinöl vorgelegt und gerührt. Anschließend werden unter Rühren die unten angegebenen Mengen Ethanol zugegeben.

| Bsp. | Talkum | Paraffinöl | Ethanol | Ergebnis |
|---|---|---|---|---|
| 1.1 | 0,1 | 5 | ad 100 | viele kleine, perlenartige Tröpfchen |
| 1.2 | 0,1 | 10 | ad 100 | relativ große, stabile perlenartige Tröpfchen |
| 1.3 | 0,3 | 20 | ad 100 | sehr schöne, gut definierte perlenarige Tröpfchen |
| 1.4 | 0,3 | 30 | ad 100 | größere, weniger gut definierte Tröpfchen |

Durch Erhöhung der Talkum-Menge kann das Verhältnis Alkohol/Paraffinöl zugunsten Öl verändert werden. Die Haarbehandlungsmittel können als haarpflegendes Tonikum verwendet werden.

### Beispiel 2: Zweiphasiges Haarbehandlungsmittel mit nahezu schwebenden perlenartigen Paraffintröpfchen

| | |
|---|---|
| 0,3 g | Talkum |
| 0,3 g | Parfüm |
| 20,0 g | Paraffinöl |
| 10,0 g | Glycerin |
| ad 100 g | Ethanol |

Das Glycerin kann ganz oder teilweise durch Wasser ersetzt werden, dem anorganische Salze wie z.B. Calciumchlorid zur Erhöhung der Dichte zugesetzt sind.

### Beispiel 3: Haarpflege-Tonikum mit abgesetzten perlenartigen Paraffintröpfchen

| | |
|---|---|
| 0,3 g | Talkum |
| 10,0 g | Paraffinöl |
| 10,0 g | Silikonöl (Dow Corning Fluid 244) |
| 10,0 g | Glycerin |
| ad 100 g | Ethanol |

### Beispiel 4: Zweiphasiges Haarbehandlungsmittel mit perlenartigen Paraffintröpfchen

| | |
|---|---|
| 0,08 g | Talkum |
| 0,08 g | Mica/Titandioxid (Timiron® Starluster MP 115) |
| 0,3 g | Parfüm |
| 5,0 g | Amphomer® (Octylacrylamide/Acrylates/Butylaminoethyl Methacrylates Copolymer) |
| 0,82 g | Aminomethylpropanol |
| 10,0 g | Paraffinöl |
| 4,7 g | Wasser |
| 4,3 g | Glycerin |
| ad 100 g | Ethanol |

### Beispiel 5: Haarpflege-Tonikum mit perlenartigen Paraffintröpfchen

| | |
|---|---|
| 0,015 g | Talkum |
| 0,015 g | Mica/Titandioxid (Timiron® Starluster MP 115) |
| 0,2 g | Polyvinylpyrrolidon |
| 10,0 g | Paraffinöl |
| 4,2 g | Wasser |
| 13,2 g | Propylenglykol |
| ad 100 g | Ethanol |

### Beispiel 6: Haarpflegemittel

| | |
|---|---|
| 0,3 g | Talkum |
| 19,5 g | Cyclomethicone (Dow Corning 245) |
| 0,2 g | Parfüm |
| 30,0 g | Ethanol |
| ad 100 g | Wasser |

Die Zusammensetzung wird in ein Behältnis abgefüllt, welches mit einer Sprühpumpe versehen ist.

### Beispiel 7: Haarpflegemittel

| | |
|---|---|
| 0,3 g | Silica (Aerosil® 300 von Degussa-Hüls) |
| 9,8 g | Isododekan |
| 9,7 g | Mineralöl (Paraffinum Perliquidum) |
| 0,2 g | Parfüm |
| ad 100 g | Ethanol |

Die Zusammensetzung wird in ein Behältnis abgefüllt, welches mit einer Sprühpumpe versehen ist.

## Patentansprüche

1. Zusammensetzung zur Behandlung von Haaren oder der Kopfhaut, enthaltend mindestens zwei nicht miteinander mischbare flüssige Phasen, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe Flüssigkeit enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm besitzen,
(C) die Zusammensetzung mindestens einen feinverteilten, pulverförmigen, in der Zusammensetzung unlöslichen, anorganischen Feststoff als Dispergator enthält,
(D) die Zusammensetzung mindestens ein filmbildendes, haarfestigendes Polymer enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet.

2. Mittel zur Behandlung von Haaren oder der Kopfhaut, bestehend aus einer Zusammensetzung und einer Vorrichtung zum Versprühen der Zusammensetzung, wobei die Zusammensetzung mindestens zwei nicht miteinander mischbare flüssige Phasen enthält, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe Flüssigkeit enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm besitzen,
(C) die Zusammensetzung mindestens einen feinverteilten, pulverförmigen, in der Zusammensetzung unlöslichen, anorganischen Feststoff als Dispergator enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet.

3. Zusammensetzung zur Behandlung von Haaren oder der Kopfhaut, enthaltend mindestens zwei nicht miteinander mischbare flüssige Phasen, wobei
(A) eine erste Phase eine kontinuierliche Phase ist und als Lösungsmittel Wasser, hydrophile organische Lösungsmittel oder ein wasserhaltiges oder wasserfreies hydrophiles Lösungsmittelgemisch enthält und wobei
(B) eine zweite Phase diskontinuierlich ist, mindestens eine hydrophobe, flüssige, leicht flüchtige Substanz mit einem Siedepunkt unter 250°C bei Normaldruck enthält und diese zweite Phase in Form von Tropfen vorliegt, welche von einer solchen Größe sind, daß die Tropfen mit bloßem Auge gut sichtbar und voneinander unterscheidbar sind, d.h. einen Durchmesser von mindestens etwa 1 mm besitzen,
(C) die Zusammensetzung mindestens einen feinverteilten, pulverförmigen, in der Zusammensetzung unlöslichen, anorganischen Feststoff als Dispergator enthält
und wobei die Zusammensetzung durch Schütteln per Hand in eine feinere Emulsion oder Pseudo-Emulsion, welche für einen für die Anwendung ausreichenden Zeitraum stabil ist, überführt werden kann und aus der sich die ursprüngliche Phasen- und Tropfenstruktur spontan und reversibel zurückbildet.

4. Zusammensetzung nach Anspruch 3, **dadurch gekennzeichnet, daß** die zweite, diskontinuierliche Phase mindestens einen leicht flüchtigen Kohlenwasserstoff und mindestens eine schwer flüchtige, hydrophobe Flüssigkeit enthält.

5. Zusammensetzung nach einem der vorangehenden Ansprüche, **dadurch gekennzeichnet, daß** das Lösungsmittel der ersten Phase ausgewählt ist aus Wasser, einwertigen verzweigten oder unverzweigten Alkoholen mit 1 bis 4 Kohlenstoffatomen und mehrwertigen Alkoholen oder Gemischen der genannten Lösungsmittel.

6. Zusammensetzung nach einem der vorangehenden Ansprüche, **dadurch gekennzeichnet, daß** die erste, kontinuierliche Phase als Lösungsmittel ein hydrophiles Lösungsmittelgemisch aus Wasser, mindestens einem einwertigen verzweigten oder unverzweigten Alkohol mit 1 bis 4 Kohlenstoffatomen und mindestens einem mehrwertigen Alkohol enthält.

7. Zusammensetzung nach Anspruch 5, **dadurch gekennzeichnet, daß** die einwertigen Alkohole ausgewählt sind aus Ethanol, n-Propanol und Isopropanol und die mehrwertigen Alkohole ausgewählt sind aus Ethylenglykol, Propylenglykol und Glycerin.

8. Zusammensetzung nach einem der vorangehenden Ansprüche, **dadurch gekennzeichnet, daß** der Dispergator ausgewählt ist aus Talkum, Calciumcarbonat, Magnesiumcarbonat, Bariumsulfat, Aluminiumhydroxid, Hydroxylapatit, Tricalciumphosphat und Calciumoxalat.

9. Zusammensetzung nach einem der vorangehenden Ansprüche, **dadurch gekennzeichnet, daß** der Dispergator in einer Menge von 0,01 bis 1 Gew.% enthalten ist.

10. Zusammensetzung nach einem der vorangehenden Ansprüche, **dadurch gekennzeichnet, daß** die hydrophobe Flüssigkeit der zweiten Phase ausgewählt ist aus pflanzlichen, synthetischen oder mineralischen, ölartigen Flüssigkeiten oder deren Gemischen.

11. Zusammensetzung nach einem der vorangehenden Ansprüche, **dadurch gekennzeichnet, daß** die hydrophobe Flüssigkeit der zweiten Phase ausgewählt ist aus Paraffinen, Isoparaffinen, Mineralöl, Paraffinöl, Pflanzenölen, Fettsäureestern, Fettsäureglyceriden, Silikonverbindungen oder deren Gemischen.

12. Zusammensetzung nach einem der vorangehenden Ansprüche, **dadurch gekennzeichnet, daß** die Dichte der beiden Phasen so eingestellt ist, daß die Tropfen der hydrophoben Phase in der hydrophilen Phase schweben oder nahezu schweben.

13. Zusammensetzung nach einem der vorangehenden Ansprüche, **dadurch gekennzeichnet, daß** die Zusammensetzung
(A) 60 bis 95 Gewichtsprozent mindestens eines einwertigen verzweigten oder unverzweigten Alkohols mit 1 bis 4 Kohlenstoffatomen,
(B) mindestens einen mehrwertigen Alkohol mit 1 bis 4 Kohlenstoffatomen in einer Menge bis maximal 30 Gewichtsprozent,
(C) Wasser in einer Menge bis maximal 30 Gewichtsprozent,
(D) 2 bis 35 Gewichtsprozent mindestens einer hydrophoben Flüssigkeit,
(E) 0,01 bis 1 Gewichtsprozent mindestens eines feinverteilten, pulverförmigen, in der Zusammensetzung unlöslichen, anorganischen Dispergators und
(F) mindestens ein filmbildendes, haarfestigendes Polymer enthält.

14. Verwendung einer Zusammensetzung gemäß einer der vorstehenden Ansprüche zur Behandlung von Haaren.

## Claims

1. Composition for treating hair or the scalp, comprising at least two immiscible liquid phases, where
(A) a first phase is a continuous phase and comprises, as solvent, water, hydrophilic organic solvents or a hydrous or anhydrous hydrophilic solvent mixture and where
(B) a second phase is discontinuous, comprises at least one hydrophobic liquid and this second phase is in the form of droplets which are of a size such that the droplets are readily visible with the naked eye and can be differentiated from one another, i.e. have a diameter of at least about 1 mm,
(C) the composition comprises at least one finely divided, pulverulent inorganic solid which is insoluble in the composition, as dispersant,
(D) the composition comprises at least one film-forming, hair-setting polymer
and where the composition can be converted, by shaking by hand, into a finer emulsion or pseudoemulsion which is stable for a period of time sufficient for the application, and from which the original phase and droplet structure reforms spontaneously and reversibly.

2. Means for treating hair or the scalp, consisting of a composition and a device for spraying the composition, where the composition comprises at least two immiscible liquid phases, where
(A) a first phase is a continuous phase and comprises, as solvent, water, hydrophilic organic solvents or a hydrous or anhydrous hydrophilic solvent mixture and where
(B) a second phase is discontinuous, comprises at least one hydrophobic liquid and this second phase is in the form of droplets which are of a size such that the droplets are readily visible with the naked eye and can be differentiated from one another, i.e. have a diameter of at least about 1 mm,
(C) the composition comprises at least one finely divided, pulverulent inorganic solid which is insoluble in the composition, as dispersant,
and where the composition can be converted, by shaking by hand, into a finer emulsion or pseudoemulsion which is stable for a period of time sufficient for the application, and from which the original phase and droplet structure reforms spontaneously and reversibly.

3. Composition for treating hair or the scalp, comprising at least two immiscible liquid phases, where
(A) a first phase is a continuous phase and comprises, as solvent, water, hydrophilic organic solvents or a hydrous or anhydrous hydrophilic solvent mixture and where
(B) a second phase is discontinuous, comprises at least one hydrophobic, liquid, readily volatile substance with a boiling point below 250°C at normal pressure, and this second phase is in the form of droplets which are of a size such that the droplets are readily visible with the naked eye and can be differentiated from one another, i.e. have a diameter of at least about 1 mm,
(C) the composition comprises at least one finely divided, pulverulent inorganic solid which is insoluble in the composition, as dispersant,
and where the composition can be converted, by shaking by hand, into a finer emulsion or pseudoemulsion which is stable for a period of time sufficient for the application, and from which the original phase and droplet structure reforms spontaneously and reversibly.

4. Composition according to Claim 3, **characterized in that** the second, discontinuous phase comprises at least one readily volatile hydrocarbon and at least one difficultly volatile hydrophobic liquid.

5. Composition according to one of the preceding claims, **characterized in that** the solvent of the first phase is chosen from water, monohydric branched or unbranched alcohols having 1 to 4 carbon atoms and polyhydric alcohols or mixtures of said solvents.

6. Composition according to one of the preceding claims, **characterized in that** the first continuous phase comprises, as solvent, a hydrophilic solvent mixture of water, at least one monohydric branched or unbranched alcohol having 1 to 4 carbon atoms and at least one polyhydric alcohol.

7. Composition according to Claim 5, **characterized in that** the monohydric alcohols are chosen from ethanol, n-propanol and isopropanol, and the polyhydric alcohols are chosen from ethylene glycol, propylene glycol and glycerol.

8. Composition according to one of the preceding claims, **characterized in that** the dispersant is chosen from talc, calcium carbonate, magnesium carbonate, barium sulphate, aluminium hydroxide, hydroxylapatite, tricalcium phosphate and calcium oxalate.

9. Composition according to one of the preceding claims, **characterized in that** the dispersant is present in an amount of from 0.01 to 1% by weight.

10. Composition according to one of the preceding claims, **characterized in that** the hydrophobic liquid of the second phase is chosen from vegetable, synthetic or mineral oily liquids or mixtures thereof.

11. Composition according to one of the preceding claims, **characterized in that** the hydrophobic liquid of the second phase is chosen from paraffins, isoparaffins, mineral oil, paraffin oil, vegetable oils, fatty acid esters, fatty acid glycerides, silicone compounds or mixtures thereof.

12. Composition according to one of the preceding claims, **characterized in that** the density of the two phases is set such that the droplets of the hydrophobic phase are suspended or virtually suspended in the hydrophilic phase.

13. Composition according to one of the preceding claims, **characterized in that** the composition comprises
(A) 60 to 95% by weight of at least one monohydric branched or unbranched alcohol having 1 to 4 carbon atoms,
(B) at least one polyhydric alcohol having 1 to 4 carbon atoms in an amount up to at most 30% by weight,
(C) water in an amount up to at most 30% by weight,
(D) 2 to 35% by weight of at least one hydrophobic liquid,
(E) 0.01 to 1% by weight of at least one finely divided, pulverulent inorganic dispersant which is insoluble in the composition and
(F) at least one film-forming, hair-setting polymer.

14. Use of a composition according to one of the claims above for treating hair.

## Revendications

1. Composition pour le traitement des cheveux ou du cuir chevelu, contenant au moins deux phases liquides non miscibles entre elles,
(A) une première phase étant une phase continue et contenant en tant que solvant de l'eau, des solvants organiques hydrophiles ou un mélange de solvants hydrophiles anhydre ou contenant de l'eau, et
(B) une seconde phase étant discontinue, contenant au moins un liquide hydrophobe, et cette seconde phase se trouvant sous forme de gouttes qui sont d'une taille telle que les gouttes sont bien visibles à l'oeil nu et peuvent être distinguées les unes des autres, c'est-à-dire ont un diamètre d'au moins environ 1 mm,
(C) la composition contenant au moins un solide minéral finement divisé, pulvérulent, insoluble dans la composition, en tant que dispersant,
(D) la composition contenant au moins un polymère filmogène fixant les cheveux,
et la composition pouvant être convertie par secouement à la main en une pseudo-émulsion ou émulsion plus fine qui est stable pendant une durée suffisante pour l'utilisation, et à partir de laquelle la structure initiale en phase et gouttes se reforme spontanément et réversiblement.

2. Produit pour le traitement des cheveux ou du cuir chevelu, constitué d'une composition et d'un dispositif pour la pulvérisation de la composition, la composition contenant au moins deux phases liquides non miscibles entre elles,
(A) une première phase étant une phase continue et contenant en tant que solvant de l'eau, des solvants organiques hydrophiles ou un mélange de solvants hydrophiles anhydre ou contenant de l'eau, et
(B) une seconde phase étant discontinue, contenant au moins un liquide hydrophobe, et cette seconde phase se trouvant sous forme de gouttes qui sont d'une taille telle que les gouttes sont bien visibles à l'oeil nu et peuvent être distinguées les unes des autres, c'est-à-dire ont un diamètre d'au moins environ 1 mm,
(C) la composition contenant au moins un solide minéral finement divisé, pulvérulent, insoluble dans la composition, en tant que dispersant, et la composition pouvant être convertie par secouement à la main en une pseudo-émulsion ou émulsion plus fine qui est stable pendant une durée suffisante pour l'utilisation, et à partir de laquelle la structure initiale en phase et gouttes se reforme spontanément et réversiblement.

3. Composition pour le traitement des cheveux ou du cuir chevelu, contenant au moins deux phases liquides non miscibles entre elles,
(A) une première phase étant une phase continue et contenant en tant que solvant de l'eau, des solvants organiques hydrophiles ou un mélange de solvants hydrophiles anhydre ou contenant de l'eau, et
(B) une seconde phase étant discontinue, contenant au moins une substance liquide hydrophobe, légèrement volatile, ayant un point d'ébullition inférieur à 250°C sous la pression normale, et cette seconde phase se trouvant sous forme de gouttes qui sont d'une taille telle que les gouttes sont bien visibles à l'oeil nu et peuvent être distinguées les unes des autres, c'est-à-dire ont un diamètre d'au moins environ 1 mm,
(C) la composition contenant au moins un solide minéral finement divisé, pulvérulent, insoluble dans la composition, en tant que dispersant, et la composition pouvant être convertie par secouement à la main en une pseudo-émulsion ou émulsion plus fine qui est stable pendant une durée suffisante pour l'utilisation, et à partir de laquelle la structure initiale en phase et gouttes se reforme spontanément et réversiblement.

4. Composition selon la revendication 3, **caractérisée en ce que** la seconde phase discontinue contient au moins un hydrocarbure aisément volatil et au moins un liquide hydrophobe peu volatil.

5. Composition selon l'une quelconque des revendications précédentes, **caractérisée en ce que** le solvant de la première phase est choisi parmi l'eau, les alcools monohydroxylés ramifiés ou non ramifiés, ayant de 1 à 4 atomes de carbone, et les alcools polyhydroxylés ou des mélanges des solvants cités.

6. Composition selon l'une quelconque des revendications précédentes, **caractérisée en ce que** la première phase continue contient en tant que solvant un mélange de solvants hydrophile à base d'eau, au moins un alcool monohydroxylé ramifié ou non ramifié, ayant de 1 à 4 atomes de carbone, et au moins un alcool polyhydroxylé.

7. Composition selon la revendication 5, **caractérisée en ce que** les alcools monohydroxylés sont choisis parmi l'éthanol, le n-propanol et l'isopropanol, et les alcools polyhydroxylés sont choisis parmi l'éthylèneglycol, le propylèneglycol et le glycérol.

8. Composition selon l'une quelconque des revendications précédentes, **caractérisée en ce que** le dispersant est choisi parmi le talc, le carbonate de calcium, le carbonate de magnésium, le sulfate de baryum, l'hydroxyde d'aluminium, l'hydroxyapatite, le phosphate tricalcique et l'oxalate de calcium.

9. Composition selon l'une quelconque des revendications précédentes, **caractérisée en ce que** le dispersant est contenu en une proportion de 0,01 à 1% en poids.

10. Composition selon l'une quelconque des revendications précédentes, **caractérisée en ce que** le liquide hydrophobe de la seconde phase est choisi parmi des liquides huileux végétaux, synthétiques ou minéraux, ou des mélanges de ceux-ci.

11. Composition selon l'une quelconque des revendications précédentes, **caractérisée en ce que** la phase liquide hydrophobe de la seconde phase est choisie parmi des paraffines, isoparaffines, une huile minérale, l'huile de paraffine, des huiles végétales, des esters d'acides gras, des glycérols d'acides gras, des composés de type silicone ou des mélanges de ceux-ci.

12. Composition selon l'une quelconque des revendications précédentes, **caractérisée en ce que** la densité des deux phases est choisie de sorte que les gouttes de la phase hydrophobe sont suspendues ou presque suspendues dans la phase hydrophile.

13. Composition selon l'une quelconque des revendications précédentes, **caractérisée en ce que** la composition contient
(A) 60 à 95% en poids d'au moins un alcool monohydroxylé, ramifié ou non ramifié, ayant de 1 à 4 atomes de carbone,
(B) au moins un alcool polyhydroxylé ayant de 1 à 4 atomes de carbone, en une quantité jusqu'à 30% en poids au maximum,
(C) de l'eau en une quantité d'au maximum 30% en poids,
(D) 2 à 35% en poids d'au moins un liquide hydrophobe,
(E) 0,01 à 1% en poids d'au moins un dispersant minéral finement divisé, pulvérulent, insoluble dans la composition, et
(F) au moins un polymère filmogène fixant les cheveux.

14. Utilisation d'une composition selon l'une quelconque des revendications précédentes, pour le traitement des cheveux.
